(19) Europäisches Patentamt
European Patent Office
Office européen des brevets

(11) EP 1 196 397 B1

# (12) EUROPEAN PATENT SPECIFICATION

(45) Date of publication and mention of the grant of the patent:
**17.08.2005 Bulletin 2005/33**

(21) Application number: **00936465.4**

(22) Date of filing: **02.06.2000**

(51) Int Cl.[7]: **C07D 241/44**, C07D 215/54, C07D 209/42, C07D 213/82, C07D 307/68, C07D 307/84, C07D 333/38, C07D 333/68, A61K 31/495, A61K 31/404, A61P 25/00

(86) International application number:
**PCT/US2000/015222**

(87) International publication number:
**WO 2000/073283 (07.12.2000 Gazette 2000/49)**

## (54) METABOTROPIC GLUTAMATE RECEPTOR ANTAGONISTS AND THEIR USE FOR TREATING CENTRAL NERVOUS SYSTEM DISEASES

METABOTROPE GLUTAMATREZEPTORANTAGONISTEN ZUR BEHANDLUNG VON KRANKHEITEN DES ZENTRALEN NERVENSYSTEMS

ANTAGONISTES DU RECEPTEUR DE GLUTAMATE METABOTROPIQUE ET LEUR UTILISATION POUR LE TRAITEMENT DE MALADIES DU SYSTEME NERVEUX CENTRAL

(84) Designated Contracting States:
**AT BE CH CY DE DK ES FI FR GB GR IE IT LI LU MC NL PT SE**

(30) Priority: **02.06.1999 US 137272 P**

(43) Date of publication of application:
**17.04.2002 Bulletin 2002/16**

(73) Proprietor: **NPS PHARMACEUTICALS, INC.**
**Salt Lake City Utah 84108 (US)**

(72) Inventors:
- **VAN WAGENEN, Bradford, C.**
  **Salt Lake City, UT 84127 (US)**
- **MOE, Scott, T.**
  **Salt Lake City, UT 84121 (US)**
- **SMITH, Daryl, L.**
  **Salt Lake City, UT 84121 (US)**
- **SHEEHAN, Susan, M.**
  **Salt Lake City, UT 84108 (US)**
- **SHCHERBAKOVA, Irina**
  **Salt Lake City, UT 84103 (US)**
- **TRAVATO, Richard**
  **Salt Lake City, UT 84117 (US)**
- **WALTON, Ruth**
  **Ogden, UT 84403 (US)**
- **BARMORE, Robert**
  **Salt Lake City, UT 84102 (US)**
- **DELMAR, Eric, G.**
  **Salt Lake City, UT 84108 (US)**
- **STORMANN, Thomas, M.**
  **Salt Lake City, UT 84105 (US)**

(74) Representative: **Plougmann & Vingtoft A/S**
**Sundkrogsgade 9,**
**P.O. Box 831**
**2100 Copenhagen O (DK)**

(56) References cited:

| | |
|---|---|
| **EP-A- 0 703 218** | **WO-A-96/05818** |
| **WO-A-99/03822** | **WO-A-99/26927** |
| **GB-A- 1 329 447** | **US-A- 5 707 985** |

- **FENG HONG ET AL.: "Design, synthesis and pharmacological test of a quinoline based, nonpeptidic analogue of neurotensin(8-13)" J. CHEM SOC. PERKIN TRANS., no. 1, 1997, pages 2083-2088, XP002101983 great britain**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention provides compounds that are active at metabotropic glutamate receptors and that are useful for treating neurological and psychiatric diseases and disorders.

### BACKGROUND OF THE INVENTION

Recent advances in the elucidation of the neurophysiological roles of metabotropic glutamate receptors have established these receptors as promising drug targets in the therapy of acute and chronic neurological and psychiatric disorders and diseases. However, the major challenge to the realization of this promise has been the development of metabotropic glutamate receptor subtypeselective compounds.

Glutamate is the major excitatory neurotransmitter in the mammalian central nervous system (CNS). Glutamate produces its effects on central neurons by binding to and thereby activating cell surface receptors. These receptors have been divided into two major classes, the ionotropic and metabotropic glutamate receptors, based on the structural features of the receptor proteins, the means by which the receptors transduce signals into the cell, and pharmacological profiles.

The metabotropic glutamate receptors (mGluRs) are G protein-coupled receptors that activate a variety of intracellular second messenger systems following the binding of glutamate. Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyl cyclase; increases in the formation of cyclic guanosine monophosphate (cGMP); activation of phospholipase $A_2$; increases in arachidonic acid release; and increases or decreases in the activity of voltage- and ligand-gated ion channels. Schoepp *et al. , Trends Pharmacol. Sci. 14*:13 (1993); Schoepp, *Neurochem. Int. 24*:439 (1994); Pin *et al., Neuropharmacology 34*:1 (1995).

Eight distinct mGluR subtypes, termed mGluR1 through mGluR8, have been identified by molecular cloning. *See, for example,* Nakanishi, *Neuron 13*:1031 (1994); Pin *et al*., *Neuropharmacology 34*:1 (1995); Knopfel *et al. , J. Med. Chem. 38*:1417 (1995). Further receptor diversity occurs via expression of alternatively spliced forms of certain mGluR subtypes. Pin *et al., PNAS 89*:10331 (1992); Minakami *et al., BBRC 199*:1136 (1994); Joly *et al., J. Neurosci. 15*:3970 (1995).

Metabotropic glutamate receptor subtypes may be subdivided into three groups, Group I, Group II, and Group III mGluRs, based on amino acid sequence homology, the second messenger systems utilized by the receptors, and by their pharmacological characteristics. Nakanishi, *Neuron 13*:1031 (1994); Pin *et al. , Neuropharmacology 34*:1 (1995); Knopfel *et al. , J. Med. Chem. 38*:1417 (1995).

Group I mGluRs comprise mGluR1, mGluR5, and their alternatively spliced variants. The binding of agonists to these receptors results in the activation of phospholipase C and the subsequent mobilization of intracellular calcium. Electrophysiological measurements have been used to demonstrate these effects in, for example, *Xenopus* oocytes expressing recombinant mGluR1 receptors. *See, for example* Masu *et al., Nature 349*:760 (1991); Pin *et al., PNAS 89*: 10331 (1992). Similar results have been achieved with oocytes expressing recombinant mGluR5 receptors. Abe *et al., J. Biol. Chem. 267*:13361 (1992); Minakami *et al., BBRC 199*:1136 (1994); Joly *et al., J. Neurosci. 15*:3970 (1995). Alternatively, agonist activation of recombinant mGluR1 receptors expressed in Chinese hamster ovary (CHO) cells stimulates PI hydrolysis, cAMP formation, and arachidonic acid release as measured by standard biochemical assays. Aramori *et al., Neuron 8*:757 (1992).

In comparison, activation of mGluR5 receptors expressed in CHO cells stimulates PI hydrolysis and subsequent intracellular calcium transients, but no stimulation of cAMP formation or arachidonic acid release is observed. Abe *et al., J. Biol. Chem. 267*:13361 (1992). However, activation of mGluR5 receptors expressed in LLC-PK1 cells results in PI hydrolysis and increased cAMP formation. Joly *et al., J. Neurosci. 15*:3970 (1995). The agonist potency profile for Group I mGluRs is quisqualate > glutamate = ibotenate > *(2S,1'S,2'S)*-2-carboxycyclopropyl)glycine (L-CCG-I) > *(1S,3R)*-1-aminocyclopentane-1,3-dicarboxylic acid (ACPD). Quisqualate is relatively selective for Group I receptors, as compared to Group II and Group III mGluRs, but it also is a potent activator of ionotropic AMPA receptors. Pin *et al. , Neuropharmacology 34*:1, Knopfel *et al., J. Med. Chem. 38*:1417 (1995).

The lack of subtype-specific mGluR agonists and antagonists has impeded elucidation of the physiological roles of particular mGluRs, and the mGluR-associated pathophysiological processes that affect the CNS have yet to be defined. However, work with the available non-specific agonists and antagonists has yielded some general insights about the Group I mGluRs as compared to the Group II and Group III mGluRs.

Attempts at elucidating the physiological roles of Group I mGluRs suggest that activation of these receptors

elicits neuronal excitation. Various studies have demonstrated that ACPD can produce postsynaptic excitation upon application to neurons in the hippocampus, cerebral cortex, cerebellum, and thalamus, as well as other brain regions. Evidence indicates that this excitation is due to direct activation of postsynaptic mGluRs, but it also has been suggested that activation of presynaptic mGluRs occurs, resulting in increased neurotransmitter release. Baskys, *Trends Pharmacol. Sci. 15*:92 (1992); Schoepp, *Neurochem. Int. 24*:439 (1994); Pin *et al., Neuropharmacology 34*:1(1995).

Pharmacological experiments implicate Group I mGluRs as the mediators of this excitatory mechanism. The effects of ACPD can be reproduced by low concentrations of quisqualate in the presence of iGluR antagonists. Hu *et al., Brain Res. 568*:339 (1991); Greene *et al., Eur. J. Pharmacol. 226*:279 (1992). Two phenylglycine compounds known to activate mGluR1, namely *(S)*-3-hydroxyphenylglycine (*(S)*-3HPG) and *(S)*-3,5-dihydroxyphenylglycine (*(S)*-DHPG), also produce excitation. Watkins *et al*., *Trends Pharmacol. Sci. 15*:33 (1994). In addition, the excitation can be blocked by *(S)*-4-carboxyphenylglycine (*(S)*-4CPG), *(S)*-4-carboxy-3-hydroxyphenylglycine (*(S)*-4C3HPG), and (+)-alpha-methyl-4-carboxyphenylglycine ((+)-MCPG), compounds known to be mGluR1 antagonists. Eaton *et al., Eur. J. Pharmacol. 244*:195 (1993); Watkins *et al*., *Trends Pharmacol. Sci. 15*:333 (1994).

Metabotropic glutamate receptors have been implicated in a number of normal processes in the mammalian CNS. Activation of mGluRs has been shown to be required for induction of hippocampal long-term potentiation and cerebellar long-term depression. Bashir *et al., Nature 363*:347 (1993); Bortolotto *et al., Nature 368*:740 (1994); Aiba *et al., Cell 79*:365 (1994); Aiba *et al., Cell 79*:377 (1994). A role for mGluR activation in nociception and analgesia also has been demonstrated. Meller *et al., Neuroreport 4*: 879 (1993). In addition, mGluR activation has been suggested to play a modulatory role in a variety of other normal processes including synaptic transmission, neuronal development, apoptotic neuronal death, synaptic plasticity, spatial learning, olfactory memory, central control of cardiac activity, waking, motor control, and control of the vestibulo-ocular reflex. For reviews, see Nakanishi, *Neuron 13*: 1031 (1994); Pin *et al., Neuropharmacology 34*:1; Knopfel *et al. , J. Med. Chem. 38*:1417 (1995).

Metabotropic glutamate receptors also have been suggested to play roles in a variety of pathophysiological processes and disease states affecting the CNS. These include stroke, head trauma, anoxic and ischemic injuries, hypoglycemia, epilepsy, and neurodegenerative diseases such as Alzheimer's disease. Schoepp *et al., Trends Pharmacol. Sci. 14*:13 (1993); Cunningham *et al., Life Sci. 54*:135 (1994); Hollman *et al., Ann. Rev. Neurosci. 17*:31 (1994); Pin *et al., Neuropharmacology 34*:1 (1995); Knopfel *et al*., *J. Med. Chem. 38*:1417 (1995). Much of the pathology in these conditions is thought to be due to excessive glutamate-induced excitation of CNS neurons. Because Group I mGluRs appear to increase glutamate-mediated neuronal excitation via postsynaptic mechanisms and enhanced presynaptic glutamate release, their activation probably contributes to the pathology. Accordingly, selective antagonists of Group I mGluR receptors could be therapeutically beneficial, specifically as neuroprotective agents or anticonvulsants.

Preliminary studies assessing therapeutic potentials with the available mGluR agonists and antagonists have yielded seemingly contradictory results. For example, it has been reported that application of ACPD onto hippocampal neurons leads to seizures and neuronal damage (Sacaan *et al., Neurosci. Lett. 139*:77 (1992); Lipparti *et al., Life Sci. 52*:85 (1993). Other studies indicate, however, that ACPD inhibits epileptiform activity, and also can exhibit neuroprotective properties. Taschenberger *et al., Neuroreport 3*:629 (1992); Sheardown, *Neuroreport 3*:916 (1992); Koh *et al., Proc. Natl. Acad. Sci. USA 88*:9431 (1991); Chiamulera *et al., Eur. J. Pharmacol. 216*:335 (1992); Siliprandi *et al*., *Eur. J. Pharmacol. 219*:173 (1992); Pizzi *et al., J. Neurochem. 61*:683 (1993).

It is likely that these conflicting results are due to the lack of selectivity of ACPD, which causes activation of several different mGluR subtypes. In the studies finding neuronal damage it appears that Group I mGluRs were activated, thereby enhancing undesirable excitatory neurotransmission. In the studies showing neuroprotective effects it appears that activation of Group II and/or Group III mGluRs occurred, inhibiting presynaptic glutamate release, and diminishing excitatory neurotransmission.

This interpretation is consistent with the observation that *(S)*-4C3HPG, a Group I mGluR antagonist and Group II mGluR agonist, protects against audiogenic seizures in DBA/2 mice, while the Group II mGluR selective agonists DCG-IV and L-CCG-I protect neurons from NMDA- and KA-induced toxicity. Thomsen *et al., J. Neurochem. 62*:2492 (1994); Bruno *et al., Eur. J. Pharmacol. 256*:109 (1994); Pizzi *et al*., *J. Neurochem. 61*:683 (1993).

Based on the foregoing, it is clear that currently available mGluR agonists and antagonists have limited value, due to their lack of potency and selectivity. Certain carboxamide compounds and others, however, are disclosed in WO 99/26927 as being useful in treating disorders associated with mGluR Group I. In addition, most currently available compounds are amino acids or amino acid derivatives that have limited bioavailabilities, thereby hampering *in vivo* studies to assess mGluR physiology, pharmacology and their therapeutic potential. Compounds that selectively inhibit activation of metabotropic glutamate receptor Group I subtypes should be useful for treatment of neurological disorders and diseases such as senile dementia, Parkinson's disease, Alzheimer's disease, Huntington's Chorea, pain, neuropathic pain, including neuropathic diseases states such as diabetic neuropathies, chemotherapy induced neuropathies, post-herpetic neuralgia, and trigeminal neuralgia epilepsy, head trauma, anoxic and ischemic injuries, psychiatric disorders and diseases such as schizophrenia, depression, and anxiety, ophthalmological disorders and diseases such

as various retinopathies, for example, diabetic retinopathies, glaucoma, and neurological disorders of a auditory nature such as tinnitus.

It is apparent, therefore, that identification of potent mGluR agonists and antagonists with high selectivity for individual mGluR subtypes, particularly for Group I receptor subtypes, are greatly to be desired.

## SUMMARY OF THE INVENTION

It is an object of the present invention, therefore, to identify metabotopic glutamate receptor-active compounds which exhibit a high degree of potency and selectivity for individual metabotropic glutamate receptor subtypes, and to provide methods of making these compounds.

It is a further object of this invention to provide pharmaceutical compositions containing compounds which exhibit a high degree of potency and selectivity for individual metabotropic glutamate receptor subtypes, and to provide methods of making these pharmaceutical compositions.

It is yet another object of this invention to provide methods of inhibiting activation of an mGluR Group I receptor, and of inhibiting neuronal damage caused by excitatory activation of an mGluR Group I receptor.

It is still another object of the invention to provide methods of treating a disease associated with glutamate-induced neuronal damage.

To accomplish these and other objectives, the present invention provides potent antagonists of Group I metabotropic glutamate receptors. These antagonists may be represented by the formula

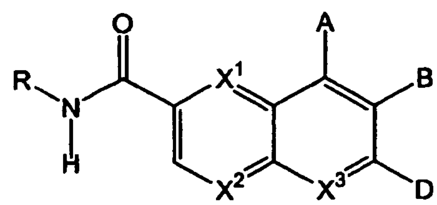

wherein $X^1$ and $X^2$ independently are CH or N,

$X^3$ is N or C-E

A, B, D, and E independently are selected from the group consisting of H, OMe, F, and $CF_3$, or B and D together are $-O-(CH_2)-O-$ or $O-(CH_2)_2-O-$, with the proviso that at least one of A, B, D and E is different from H,

R is selected from the group consisting of:

$C_4$-$C_6$ alkyl,

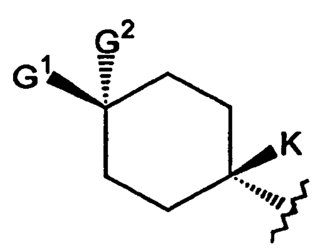

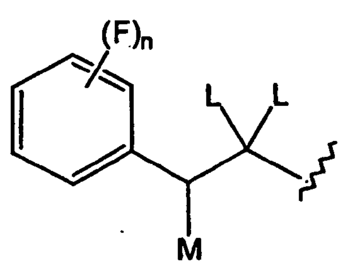 , 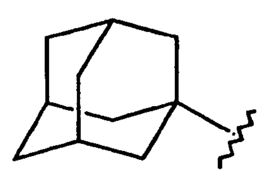 ,

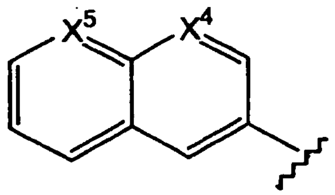 and 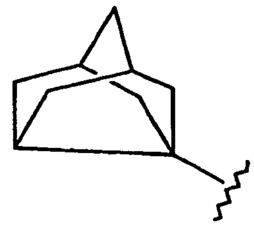 ,

wherein K is H or Me,

$G^1$ is H, Me, or phenyl,

$G^{2,}$ K, L, and M independently are H or Me,

n is 0, 1 or 2,

and $X^4$ and $X^5$ independently are N or CH, or a pharmaceutically acceptable salt thereof.

The compound is selected from the group consisting of *N*-(*trans*-4-methylcyclohexyl)-6-methoxyquinoline-3-carboxamide, *N*-(*trans*-4-methylcyclohexyl)-7-methoxyquinoline-3-carboxamide, *N*-(*trans*-4-methylcyclohexyl)-5-fluoroquinoline-3-carboxamide, *N*-(*trans*-4-methylcyclohexyl)-7-fluoroquinoline-3-carboxamide, *N*-(*trans*-4-methylcyclohexyl)-6-fluoroquinoline-3-carboxamide, *N*-(*trans*-4-methylcyclohexyl)-8-fluoroquinoline-3-carboxamide, *N*-(*trans*-4-methylcyclohexyl)-6,7-methylenedioxyquinoline-3-carboxamide, *N*-(*trans*-4-methylcyclohexyl)-6,7-ethylenedioxyquinoline-3-carboxamide, *N*-(*trans*-4-methylcyclohexyl)-6,8-difluoroquinoline-3-carboxamide, *N*-(*trans*-4-methylcyclohexyl)-6-methoxy-7-fluoroquinoline-3-carboxamide, *N*-(*trans*-4-methylcyclohexyl)-7-trifluoromethylquinoline-3-carboxamide, *N*-(*trans*-4-methylcyclohexyl)-8-trifluoromethylquinoline-3-carboxamide, *N*-(*trans*-4-methylcyclohexyl)-6-fluoroquinoxaline-2-carboxamide, *N*-(*trans*-4-methylcyclohexyl)-5-fluoroquinoxaline-2-carboxamide, *N*-(*trans*-4-methylcyclohexyl)-6-trifluoromethylquinoxaline-2-carboxamide, *N*-(*trans*-4-methylcyclohexyl)-6-methoxyquinoxaline-2-carboxamide, *N*-(*trans*-4-methylcyclohexyl)-6,7-methylenedioxyquinoxaline-2-carboxamide, *N*-(*trans*-4-methylcyclohexyl)-8-methoxyquinoline-3-carboxamide, *N*-(*trans*-4-methylcyclohexyl)-5,7-difluoroquinoline-3-carboxamide, *N*-(*trans*-4-methylcyclohexyl)-6-trifluoromethylquinoline-3-carboxamide, and pharmaceutically acceptable salts thereof.

In a preferred embodiment, the compound is selected from the group consisting of *N*-(*trans*-4-methylcyclohexyl)-6-methoxyquinoline-3-carboxamide, *N*-(*trans*-4-methylcyclohexyl)-7-methoxyquinoline-3-carboxamide, *N*-(*trans*-4-methylcyclohexyl)-5-fluoroquinoline-3-carboxamide, *N*-(*trans*-4-methylcyclohexyl)-7-fluoroquinoline-3-carboxamide, *N*-(*trans*-4-methylcyclohexyl)-6-fluoroquinoline-3-carboxamide, *N*-(*trans*-4-methylcyclohexyl)-8-fluoroquinoline-3-carboxamide, *N*-(*trans*-4-methylcyclohexyl)-6,7-methylenedioxyquinoline-3-carboxamide, *N*-(*trans*-4-methylcyclohexyl)-6,7-ethylenedioxyquinoline-3-carboxamide, *N*-(*trans*-4-methylcyclohexyl)-6,8-difluoroquinoline-3-carboxamide, *N*-(*trans*-4-methylcyclohexyl)-6-methoxy-7-fluoroquinoline-3-carboxamide, *N*-(*trans*-4-methylcyclohexyl)-7-trifluoromethylquinoline-3-carboxamide, *N*-(*trans*-4-methylcyclohexyl)-8-trifluoromethylquinoline-3-carboxamide, *N*-(*trans*-4-methylcyclohexyl)-6-fluoroquinoxaline-2-carboxamide, *N*-(*trans*-4-methylcyclohexyl)-5-fluoroquinoxaline-2-carboxamide, *N*-(*trans*-4-methylcyclohexyl)-6-trifluoromethylquinoxaline-2-carboxamide, *N*-(*trans*-4-methylcyclohexyl)-6-methoxyquinoxaline-2-carboxamide, *N*-(*trans*-4-methylcyclohexyl)-6,7-methylenedioxyquinoxaline-2-carboxamide, *N*-(*trans*-4-methylcyclohexyl)-8-methoxyquinoline-3-carboxamide, *N*-(*trans*-4-methylcyclohexyl)-5,7-difluoroquinoline-3-carboxamide, and *N*-(*trans*-4-methylcyclohexyl)-6-trifluoromethylquinoline-3-carboxamide, and pharmaceutically acceptable salts thereof.

In another preferred embodiment, the compound is selected from the group consisting of *N*-(*trans*-4-methylcyclohexyl)-6-methoxyquinoline-3-carboxamide, *N*-(*trans*-4-methylcyclohexyl)-7-methoxyquinoline-3-carboxamide, *N*-(*trans*-4-methylcyclohexyl)-5-fluoroquinoline-3-carboxamide, *N*-(*trans*-4-methylcyclohexyl)-7-fluoroquinoline-3-carboxamide, *N*-(*trans*-4-methylcyclohexyl)-6-fluoroquinoline-3-carboxamide, *N*-(*trans*-4-methylcyclohexyl)-8-fluoroquinoline-3-carboxamide, *N*-(*trans*-4-methylcyclohexyl)-6,7-methylenedioxyquinoline-3-carboxamide, *N*-(*trans*-4-methylcyclohexyl)-6,7-ethylenedioxyquinoline-3-carboxamide, *N*-(*trans*-4-methylcyclohexyl)-6,8-difluoroquinoline-3-carboxamide, *N*-(*trans*-4-methylcyclohexyl)-6-methoxy-7-fluoroquinoline-3-carboxamide, *N*-(*trans*-4-methylcyclohexyl)-7-trifluoromethylquinoline-3-carboxamide, *N*-(*trans*-4-methylcyclohexyl)-8-trifluoromethylquinoline-3-carboxamide, *N*-(*trans*-4-methylcyclohexyl)-6-fluoroquinoxaline-2-carboxamide, *N*-(*trans*-4-methylcyclohexyl)-5-fluoroquinoxaline-2-carboxamide, *N*-(*trans*-4-methylcyclohexyl)-6-trifluoromethylquinoxaline-2-carboxamide, *N*-(*trans*-4-methylcyclohexyl)-6-methoxyquinoxaline-2-carboxamide, and *N*-(*trans*-4-methylcyclohexyl)-6,7-methylenedioxyquinoxaline-2-carboxamide, and pharmaceutically acceptable salts thereof.

In accordance with another embodiment of the invention, there has been provided a pharmaceutical composition comprising a compound as set forth above, together with a pharmaceutically acceptable diluent or excipient.

In accordance with still another embodiment of the invention, there has been provided a method of making a compound as set forth above, comprising reacting a compound containing an activated carboxylic acid group with a

compound containing an amine, hydroxyl, or thiol group.

In accordance with a still further embodiment of the invention, there has been provided a method of inhibiting activation of an mGluR Group I receptor, comprising treating a cell containing said mGluR Group I receptor with an effective amount of a compound as set forth above.

In yet another embodiment of the invention, there has been provided a method of inhibiting neuronal damage caused by excitatory activation of an mGluR Group I receptor, comprising treating neurons with an effective amount of a compound as set forth above.

In accordance with a further embodiment of the invention, there has been provided a method of treating a disease associated with glutamate-induced neuronal damage, comprising administering to a patient suffering from said disease an effective amount of a composition as set forth above.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

## BRIEF DESCRIPTION OF THE DRAWINGS

Figure 1 shows illustrative compounds of the invention.

## DETAILED DESCRIPTION

The invention provides compounds that are potent and selective antagonists of Group I metabovopic glutamate receptors. The compounds contemplated by the invention can be represented by the general formula

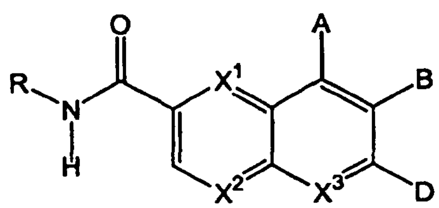

wherein $X^1$ and $X^2$ independently are CH or N,

$X^3$ is N or C-E

A, B, D, and E independently are selected from the group consisting of H, OMe, F, and $CF_3$, or B and D together are -O-($CH_2$)-O- or O-$(CH_2)_2$-O-, with the proviso that at least one of A, B, D and E is different from H,

R is selected from the group consisting of:

$C_4$-$C_6$ alkyl,

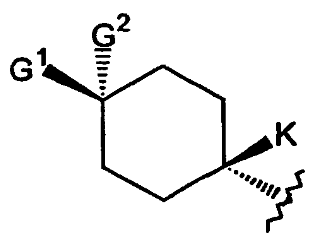

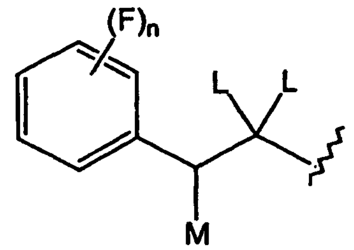 , 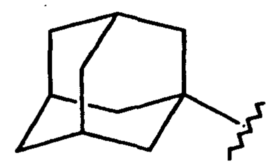 ,

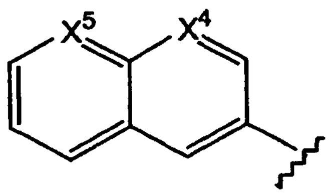 and 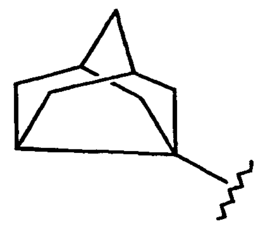 ,

wherein K is H or Me,

$G^1$ is H, Me, or phenyl,

$G^2$, K, L, and M independently are H or Me,

n is 0, 1 or 2,

and $X^4$ and $X^5$ independently are N or CH, or a pharmaceutically acceptable salt thereof.

When compounds may be present in alternative isomeric configurations, for example, *trans* or *cis*-4-methylcyclohexyl, the R moiety may have any of the possible configurations. Similarly, if a compound exists as enantiomers, the R moiety can be either of the enantiomers, or may be a racemate.

In each of the compounds described above, "alkyl" denotes both straight and branched chain alkyl.

The skilled artisan also will recognize that the compounds of the invention encompass salts of the compounds described above. These salts include pharmaceutically acceptable acid addition salts, pharmaceutically acceptable metal salts or optionally alkylated ammonium salts, such as hydrochloric, hydrobromic, hydroiodic, phosphoric, sulfuric, trifluoroacetic, malonic, succinic, citric, mandelic, benzoic, cinnamic, methanesulfonic and similar ones, and include acids related to the pharmaceutically acceptable salts listed in the *Journal of Pharmaceutical Sciences*, 66:2 (1977) and incorporated herein by reference.

Examples of compounds according to the present invention are set forth in Table 1 below.

**Preparation of mGluR Group I antagonists**

The skilled artisan will recognize that mGluR Group I antagonists according to the invention may be prepared by methods that are well known in the art, using widely recognized techniques of organic chemistry. Suitable reactions are described in standard textbooks of organic chemistry. For example, see March, Advanced Organic Chemistry, 2d ed., McGraw Hill (1977).

The carboxamide compounds generally may be prepared using well known techniques, such as reaction between an amine and an acid chloride, or by reaction in the presence of a coupling reagent such as carbonyldiimidazole, or a carbodiimide such as, for example, 1,3-dicyclohexylcarbodiimide (DCC). Formation of ester and thioester linkages can be achieved in similar fashion.

In most cases, the precursor moieties are readily available, or may be prepared using straightforward techniques of organic chemistry. Many compounds are commercially available, for example, from Aldrich Chemical Company, Milwaukee, WI. When the compounds are not commercially available, they may readily prepared from available precursors using straightforward transformations that are well known in the art.

For example, carboxylic acids may be converted into the corresponding acid chlorides by reaction with, for example, thionyl chloride or oxalyl chloride. An example of such a reaction is provided below in Example 3. Compounds containing a hydroxy function may be converted into the corresponding amine by (i) conversion of the hydroxyl group into a leaving group, such as a sulfonic acid ester (such as a triflate, mesylate, or tosylate) or a halide, (ii) displacement with azide ion, and (iii) reduction of the resulting azide by, for example, hydrogenation over a platinum oxide catalyst. An illustration of such a transformation is provided below in Example 12.

### Testing of compounds for mGluR Group I antagonist activity

The pharmacological properties of the compounds of the invention can be analyzed using standard assays for functional activity. Examples of glutamate receptor assays are well known in the art, for example, see Aramori *et al., Neuron* 8:757 (1992); Tanabe *et* al., *Neuron* 8:169 (1992). The methodology described in those publications is incorporated herein by reference.
Conveniently, the compounds of the invention may be studied using an assay that measures inhibition of intracellular calcium mobilization in cells expressing recombinant receptors that can bind the compounds. Suitable receptor constructs are well known in the art and are also described, for example, in WO 97/05252, the contents of which are hereby incorporated by reference in their entirety.
Thus, HEK-293 cells (human embryonic kidney cells, available from the American Type Culture Collection, Rockville, MD, Accession Number CRL 1573) are stably transfected with a DNA construct expressing a recombinant receptor. The stably transfected cells are cultured in high glucose DMEM (Gibco 092) containing 0.8 mM glutamine, 10% FBS, and 200 μM hygromycin B.
A protocol for measuring intracellular calcium mobilization in response to changes in extracellular calcium using the calcium-sensitive dye Fura has been described previously. Briefly, HEK-293 cells, stably transfected with a DNA construct encoding a recombinant receptor, are loaded with Fura dye. The cells then are washed, resuspended, and maintained at 37 °C. The cells are diluted into cuvettes for recording fluorescent signals. Measurements of fluorescence are performed at 37 °C using standard methods, and concentrations of intracellular $Ca^{2+}$ are calculated using a dissociation constant (Kd) of 224 nM and applying equation:

$$[Ca^{2+}]_i = (F - F_{min} / F_{max}) \times Kd$$

where F is fluorescence at any particular time of interest, $F_{min}$ is determined by chelating all calcium available, therefore, no fura 2 is bound to calcium, and $F_{max}$ is determined by fully saturating all the fura 2 available with calcium.
A detailed protocol for testing the compounds of the invention is provided below at Example 15.

### Preparation of pharmaceutical compositions containing mGluR antagonists, and their use in treating neurological disorders

The compounds of the invention are useful for treating neurological disorders or diseases. While these compounds will typically be used in therapy for human patients, they may also be used in veterinary medicine to treat similar or identical diseases.
In therapeutic and/or diagnostic applications, the compounds of the invention can be formulated for a variety of modes of administration, including systemic and topical or localized administration. Techniques and formulations generally may be found in Remington's Pharmaceutical Sciences: Drug Receptors and Receptor Theory, 18th ed., Mack Publishing Co. (1990).
The compounds according to the invention are effective over a wide dosage range. For example, in the treatment of adult humans, dosages from about 0.01 to about 1000 mg, preferably from about 0.5 to about 100 mg, per day may be used. A most preferable dosage is about 2 mg to about 70 mg per day. The exact dosage will depend upon the route of administration, the form in which the compound is administered, the subject to be treated, the body weight of the subject to be treated, and the preference and experience of the attending physician.
Pharmaceutically acceptable salts are generally well known to those of ordinary skill in the art, and may include, by way of example but not limitation, acetate, benzenesulfonate, besylate, benzoate, bicarbonate, bitartrate, bromide, calcium edetate, camsylate, carbonate, citrate, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, mucate, napsylate, nitrate, pamoate (embonate), pantothenate, phosphate/diphosphate, polygalacturonate, salicylate, stearate, subacetate, succinate, sulfate, tannate, tartrate, or teoclate. Other pharmaceutically acceptable salts may be found in, for example, Remington's Pharmaceutical Sciences; (18th ed.), Mack Publishing Co., Easton,PA (1990).
Preferred pharmaceutically acceptable salts include, for example, acetate, benzoate, bromide, carbonate, citrate, gluconate, hydrobromide, hydrochloride, maleate, mesylate, napsylate, pamoate (embonate), phosphate, salicylate, succinate, sulfate, or tartrate.
Depending on the specific conditions being treated, such agents may be formulated into liquid or solid dosage forms and administered systemically or locally. The agents may be delivered, for example, in a timed- or sustained-release form as is known to those skilled in the art. Techniques for formulation and administration may be found in Remington's Pharmaceutical Sciences; (18th ed.), Mack Publishing Co., Easton, PA (1990). Suitable routes may include oral, buccal, sublingual, rectal, transdermal, vaginal, transmucosal, nasal or intestinal administration; parenteral delivery, including intramuscular, subcutaneous, intramedullary injections, as well as intrathecal, direct intraventricular, intravenous, intraperitoneal, intranasal, or intraocular injections, just to name a few.

For injection, the agents of the invention may be formulated in aqueous solutions, preferably in physiologically compatible buffers such as Hank's solution, Ringer's solution, or physiological saline buffer. For such transmucosal administration, penetrants appropriate to the barrier to be permeated are used in the formulation. Such penetrants are generally known in the art.

Use of pharmaceutically acceptable carriers to formulate the compounds herein disclosed for the practice of the invention into dosages suitable for systemic administration is within the scope of the invention. With proper choice of carrier and suitable manufacturing practice, the compositions of the present invention, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the invention to be formulated as tablets, pills, capsules, liquids, gels, syrups, slurries, suspensions and the like, for oral ingestion by a patient to be treated.

Pharmaceutical compositions suitable for use in the present invention include compositions wherein the active ingredients are contained in an effective amount to achieve its intended purpose. Determination of the effective amounts is well within the capability of those skilled in the art, especially in light of the detailed disclosure provided herein.

In addition to the active ingredients, these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions.

Pharmaceutical preparations for oral use can be obtained by combining the active compounds with solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations, for example, maize starch, wheat starch, rice starch, potato starch, gelatin, gum tragacanth, methyl cellulose, hydroxypropylmethyl-cellulose, sodium carboxymethyl-cellulose (CMC), and/or polyvinylpyrrolidone (PVP: povidone). If desired, disintegrating agents may be added, such as the cross-linked polyvinylpyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinylpyrrolidone, carbopol gel, polyethylene glycol (PEG), and/or titanium dioxide, lacquer solutions, and suitable organic solvents or solvent mixtures. Dye-stuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin, and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols (PEGs). In addition, stabilizers may be added.

The present invention, thus generally described, will be understood more readily by reference to the following examples, which are provided by way of illustration and are not intended to be limiting of the present invention. Examples 6-10 are reference examples not encompassed by the claimed invention.

## EXAMPLES

### General Experimental Methods

Capillary gas chromatographic and mass spectral data were obtained using a Hewlett-Packard (HP) 5890 Series II Gas Chromatograph coupled to an HP 5971 Series Mass Selective Detector [Ultra-2 Ultra Performance Capillary Column (crosslinked 5% PhMe silicone); column length, 25 m; column i.d., 0.20 mm; helium flow rate, 60 mL/min; injector temp., 250 °C; temperature program, 20 C/min from 125 to 325 °C for 10 min, then held constant at 325 °C for 6 min]. Thin-layer chromatography was performed using Analtech Uniplate 250-µm silica gel HF TLC plates. UV light sometimes in conjunction with ninhydrin and Dragendorff's spray reagents (Sigma Chemical Co.) were used for detecting compounds on the TLC plates. Reagents used in reactions were purchased from the Aldrich Chemical Co. (Milwaukee, WI), Sigma Chemical Co. (Saint Louis, MO), Fluka Chemical Corp. (Milwaukee, WI), Fisher Scientific (Pittsburgh, PA), TCI America (Portland, OR), or Lancaster Synthesis (Windham, NH).

**EXAMPLE 6 (Reference): Preparation of *N*-(*trans*-4-methylcyclohexyl)-6-(1-pyrazole)nicotinamide (408)**

A solution of 6-(1-pyrazole)nicotinic acid (96 mg, 0.51 mmol), and 1,1'-carbonyldiimidazole (83 mg, 0.51 mmol) in dimethylformamide (2 mL) was heated at 50 °C for 1 hour. After this time, *trans*-4-methylcyclohexylamine hydrochloride (76 mg, 0.51 mmol), and *N,N*-diisopropylethylamine (0.135 mL, 0.77 mmol) were added and the mixture heated at 50 °C for 16 hours. The reaction mixture was cooled, and diluted with chloroform (10 mL). The organic solution was washed with water (4 x 10 mL), brine (10 mL), dried over anhydrous $MgSO_4$, filtered and concentrated to afford 75 mg (52 %) of 408: rt=10.5 min.; m/z (rel. int.) 284 ($M^+$, 18), 189 (48), 172 (100), 144 (13), 117 (23), 90 (10).

**EXAMPLE 7 (Reference): Preparation of *N*-(*trans*-4-methylcyclohexyl)-6-bromopicolinamide (409)**

A solution of 6-bromopicolinic acid (3 g, 14.85 mmol), and 1,1'-carbonyldiimidazole (2.41 g, 14.85 mmol) in dimethylformamide (30 mL) was heated at 50 °C for 2 hours. After this time, *trans*-4-methylcyclohexylamine hydrochloride (2.2 g, 14.85 mmol), and *N,N*-diisopropylethylamine (3.88 mL, 22.3 mmol) were added and the mixture heated at 50 °C for 16 hours. The reaction mixture was cooled, and diluted with ethyl acetate (300 mL). The organic solution was washed with water (4 x 300 mL). The aqueous extracts were washed with ethyl acetate (2 x 250 mL). The combined organic extracts were dried over anhydrous $MgSO_4$, filtered and concentrated. Chromatography of the crude product through a Biotage silica cartridge (15 x 4 cm i.d.) using ethyl acetate - hexane (1:3, containing 1% diethylamine) afforded 3.84 g (87%) of 409:
rt=8.36 min.; m/z (rel. int.) 298 ($M^+$, 10), 296 (M+, 10), 239 (28), 241 (28), 201 (38), 203 (38), 184 (44), 186 (44), 158 (47), 156 (47), 112 (100).

**EXAMPLE 8 (Reference): Preparation of *N*-(*trans*-4-methylcyclohexyl)-6-(phenoxy)picolinamide (410)**

A nitrogen purged mixture of *N*-(*trans*-4-methylcyclohexyl)-6-bromo picolinamide (446 mg, 1.5 mmol), sodium phenoxide (209 mg, 1.8 mmol), and [1,1'-bis(diphenylphosphino)ferrocene]dichloropalladium(II) (1:1 complex with dichloromethane; 83 mg, 0.1 mmol) in toluene - tetrahydrofuran (9:1, 5 mL) was treated with bis(dibenzylideneacetone) palladium(II) (86 mg, 0.15 mmol) and the mixture heated at reflux overnight. After this time the reaction mixture was diluted with ethyl acetate (25 mL) and filtered. The organic solution was washed with water (2 x 20 mL), brine, dried over anhydrous $MgSO_4$, filtered and concentrated. Chromatography of the crude product on silica (2 mm, chromatotron) with ethyl acetate - hexane (1:3, containing 1% diethylamine) afforded 97 mg (21%) of 410:
rt=8.36 min.; m/z (rel. int.) 310 ($M^+$, 31), 265 (M+, 33), 253 (28), 239 (6), 215 (30), 198 (33), 185 (16), 170 (100), 112 (75), 77 (47).

**EXAMPLE 9 (Reference): Preparation of *N*-(adamantyl)-5-(1-piperidine)nicotinamide (411)**

In a sealed tube, a solution of *N*-(adamantyl)-5-bromonicotinamide (335 mg 1 mmol), piperidine (2 mL), and 1,8-diazabicyclo[5.4.0]undec-7-ene (1 mL, 6.7 mmol) was heated at 200 °C for 5 days. Workup and chromatography through a Biotage silica cartridge (8 x 4 cm i.d.) using a gradient of chloroform to 5% methanol in chloroform afforded 10 mg (3%) of 411:
rt=12.8 min.; m/z (rel. int.) 339 ($M^+$, 100), 298 (3), 282 (15), 204 (10); 189 (12), 161 (15).

**EXAMPLE 10 (Reference): Preparation of *N*-(*trans*-4-methylcyclohexyl) benzothiazole-2-carboxamide (412)**

Using the method of Skraup (Chem. Ber., 1922, 55, 1089-1090), a solution of potassium permaganate (4.75 g, 30 mmol) in water (100 mL) was treated with 2-methylbenzothiazole (2.2 g, 15 mmol) and the mixture heated at reflux for 2.5 hours. After this time the reaction was filtered hot and the filtrate allowed to cool. The solution was then extracted once with diethyl ether (200 mL) and the remaining aqueous phase acidified (pH 1) by the addition of concentrated HCl. The aqueous phase was then extracted with dichloromethane (1 x 200 mL, 2 x 100 mL). The combined organic extracts were dried over anhydrous $MgSO_4$, filtered and concentrated to afford 400 mg (15%) of benzothiazole-2-carboxylic acid.

A solution of benzothiazole-2-carboxylic acid (359 mg, 2 mmol), and 1,1'-carbonyldiimidazole (325 mg, 2 mmol) in dimethylformamide (4 mL) was heated at 50 °C for 1 hour. After this time, *trans*-4-methylcyclohexylamine hydrochloride (300 mg, 2 mmol), and *N,N*-diisopropylethylamine (0.525 mL, 3 mmol) were added and the mixture heated at 50 °C for 3 hours. The reaction mixture was cooled, and treated with 10% HCl (15 mL). The solution was extracted with ethyl acetate (15 mL). The ethyl acetate extract was then washed with 10% HCl (15 mL), water (15 mL), and brine (15 mL). Dilution of the remaining solution with ethyl acetate (50 mL) and cooling to -20 °C afforded 25 mg (5 %) 412:

rt=9.62 min.; m/z (rel. int.) 274 ($M^+$, 43), 229 (8), 217 (29), 203 (18), 179 (29), 162 (100), 135 (69), 134 (67), 112 (53).

**EXAMPLE 17: Preparation of *N*-(*trans*-4-methylcyclohexyl)-6-methoxyquinoline-3-carboxamide (419)**

6-Methoxyquinoline-3-carboxylic acid was prepared from *p*-anisidine using the methods of Erickson (J.Med. Chem.,1979, 22, 7, 816-823). A solution of 6-methoxyquinoline-3-carboxylic acid hydrochloride (500 mg, 2.09 mmol) in thionyl chloride (25 mL) was heated at reflux for 1 hour. After this time the excess thionyl chloride was removed by distillation and the remaining solid dried under vacuum. The solid was suspended in dichloromethane (25 mL) and treated with *trans*-4-methylcyclohexylamine hydrochloride (368 mg, 2.46 mmol), pyridine (5 mL), and triethylamine (0.345 mL). The reaction was stirred at ambient temperature overnight. After this time the reaction mixture was diluted with diethyl ether (200 mL) and the resulting organic solution washed with 1 *N* NaOH (3 x 50 mL) and then brine (1x). The organic solution was then dried over anhydrous $MgSO_4$, filtered, and concentrated to a solid. Chromatography of the crude product through a Biotage silica cartridge (8 x 4 cm i.d.) using a gradient of hexane to 50% ethyl acetate (in hexane) afforded 100 mg (16%) of 419:
rt=10.95 min.; m/z (rel. int.) 298 ($M^+$, 3), 202 (56), 186 (66), 158 (100), 143 (16), 116 (34), 101 (22), 88 (25), 77 (45), 55 (44), 41 (85).
In a similar manner, the following substituted quinoline-3-carboxamides were prepared:

**EXAMPLE 18: Preparation of *N*-(*trans*-4-methylcyclohexyl)-7-methoxyquinoline-3-carboxamide (420)**

7-Methoxyquinoline-3-carboxylic acid hydrochloride (302 mg, 1.26 mmol) and *trans*-4-methylcyclohexylamine hydrochloride (188 mg, 1.26 mmol) afforded crude product. Chromatography of the crude product through a Biotage silica cartridge (8 x 4 cm i.d.) using a gradient of hexane to 50% ethyl acetate (in hexane) afforded 21 mg of semi-pure product. Recrystallization (2x) from hot toluene afforded 4 mg (1%) of 420:
rt=10.95 min.; m/z (rel. int.) 298 ($M^+$, 2), 202 (73), 186 (100), 158 (87), 143 (11), 131 (16), 116 (42), 101 (24), 88 (32), 77 (54), 55 (54), 41 (97).

**EXAMPLE 19: Preparation of *N*-(*trans*-4-methylcyclohexyl)-8-methoxyquinoline-3-carboxamide (421)**

8-Methoxyquinoline-3-carboxylic acid hydrochloride (250 mg, 1.04 mmol), oxalyl chloride, and *trans*-4-methylcyclohexylamine hydrochloride (156 mg, 1.04 mmol) afforded crude product. Chromatography of the crude product through a Biotage silica cartridge (8 x 4 cm i.d.) using a gradient of hexane to 75% ethyl acetate (in hexane) afforded 52 mg (17%) of 421:
rt=10.95 min.; m/z (rel. int.) 298 ($M^+$, 100), 269 (24), 201 (63), 186 (91), 158 (48), 128 (39), 116 (12), 101 (11), 88 (5), 77 (7), 55 (5), 41 (8).

**EXAMPLE 20: Preparation of *N*-(*trans*-4-methylcyclohexyl)-5-fluoroquinoline-3-carboxamide (422) and *N*-(*trans*-4-methylcyclohexyl)-7-fluoroquinoline-3-carboxamide (423)**

The methods of Erickson (J.Med.Chem.,1979, 22, 7, 816-823) were used to prepare a mixture of 5-fluoroquinoline-3-carboxylic acid hydrochloride and 7-fluoroquinoline-3-carboxylic acid hydrochloride from *m*-fluoroaniline. A mixture of these two acids (500 mg, 2.2 mmole) in dichloromethane (25 mL) was treated with a solution of oxalyl chloride in dichloromethane (1.2 mL of 2 M, 2,4 mmol) followed by dimethylformamide (5 drops). The reaction was stirred overnight, filtered (Gelman Acrodisc CR PTFE 0.45 micron) and concentrated to a solid. The solid was dissolved in dichloromethane (25 mL) and then treated with *trans*-4-methylcyclohexylamine hydrochloride (330 mg, 2.2 mmol) followed by 4-*N,N*'-dimethylaminopyridine (1.22 g, 10 mmol). The reaction was stirred 1 hour at ambient temperature. Chromatography of the crude reaction mixture through a Biotage silica cartridge (8 x 4 cm i.d.) using a gradient of hexane to 30% ethyl acetate (in hexane) afforded three fractions. Recrystallization of fraction A from hot heptane afforded 30 mg of *N*-(*trans*-4-methylcyclohexyl)-7-fluoroquinoline-3-carboxamide (423):
rt=9.62 min.; m/z (rel. int.) 286 ($M^+$, 18), 191 (97), 174 (100), 146 (77), 126 (12), 119 (20), 99 (10), 81 (10).
Recrystallization of fraction C from hot heptane (2x) and heptane/dichloromethane (1x) afforded 8 mg of *N*-(*trans*-4-methyleyclohexyl)-5-fluoroquinoline-3-carboxamide (422):
rt=9.54 min.; m/z (rel. int.) 286 ($M^+$, 15), 191 (95), 174 (100), 146 (80), 126 (20), 119 (23), 99 (12), 81 (12).

**EXAMPLE 21: Preparation of *N*-(*trans*-4-methylcyclohexyl)-6-fluoroquinoline-3-carboxamide (424)**

6-Fluoroquinoline-3-carboxylic acid hydrochloride (500 mg, 2.2 mmol), oxalyl chloride, and *trans*-4-methylcyclohexylamine hydrochloride (330 mg, 2.2 mmol) afforded crude product. Chromatography through a Biotage silica

cartridge (8 x 4 cm i.d.) using a gradient of hexane to 40% ethyl acetate (in hexane) afforded 56 mg (9%) of 424: rt=9.54 min.; m/z (rel. int.) 286 ($M^+$, 12), 191 (100), 174 (88), 146 (83), 126 (13), 119 (19), 99 (9), 81 (11).

**EXAMPLE 22: Preparation of *N*-(*trans*-4-methylcyclohexyl)-8-fluoroquinoline-3-carboxamide (425)**

8-Fluoroquinoline-3-carboxylic acid hydrochloride (500 mg, 2.2 mmol), oxalyl chloride, and *trans*-4-methylcyclohexylamine hydrochloride (330 mg, 2.2 mmol) afforded crude product. Chromatography through a Biotage silica cartridge (8 x 4 cm i.d.) using a gradient of hexane to 40% ethyl acetate (in hexane) afforded 81 mg (13%) of 425: rt = 10.12 min.; m/z (rel. int.) 286 ($M^+$, 17), 191 (100), 174 (83), 146 (67), 126 (20), 119 (7), 99 (7), 81 (7).

**EXAMPLE 23: Preparation of *N*-(*trans*-4-methylcyclohexyl)-6,7-methylenedioxyquinoline-3-carboxamide (426)**

Ethyl 4-chloro-6,7-methylenedioxyquinoline-3-carboxylate was prepared from 3,4-(methylenedioxy)aniline using the methods of Erickson (J.Med.Chem.,1979, 22, 7, 816-823).

Ethyl 4-chloro-6,7-methylenedioxyquinoline-3-carboxylate (18.5 g, 66.2 mmol) was dissolved in dioxane (100 mL) and treated with 50 mL 5 *N* NaOH and heated at reflux for 2 hour. After this time the solution was concentrated on a rotary evaporator, cooled and extracted with dichloromethane (2 x 100 mL). The remaining aqueous solution was then acidified with concentrated HCl and the precipitate collected and dried to afford 7 g of crude 4-hydroxy-6,7-methylenedioxyquinoline-3-carboxylic acid hydrochloride.

The crude acid (500 mg) was treated with thionyl chloride (20 mL) and heated at reflux for 1 hour. After this time the excess thionyl chloride was removed by distillation and the remaining solid pumped dry. The solid was dissolved in dichloromethane and treated with *trans*-4-methylcyclohexylamine hydrochloride (150 mg, 1 mmol), followed by triethylamine (0.3 mLs). The reaction was stirred 30 min and concentrated. The crude reaction mixture was partitioned between water (50 mL) and dichloromethane (4 x 25 mL). The combined dichloromethane extracts, were dried over anhydrous $MgSO_4$, filtered and concentrated to afford crude *N*-(*trans*-4-methylcyclohexyl)-4-chloro-6,7-methylenedioxyquinoline-3-carboxamide.

Without purification the amide was dissolved in hot toluene (25 mL) and diluted with ethanol (100 mL). *p*-Toluene sulfonic acid (400 mg) and 10% Pd on carbon (200 mg) were added and the reaction shook under 60 p.s.i. $H_2$, for 75 min at ambient temperature. The reaction was filtered, washing with ethanol and chloroform. The solvents were evaporated and the remaining solid dissolved in dichloromethane (50 mL) and equilibrated with 1 *N* NaOH. The organic layer was removed and the remaining aqueous phase extracted an additional two times with dichloromethane (2 x 50 mL). The combined organic washes were dried over anhydrous $MgSO_4$, filtered and concentrated to a solid (352 mg). Chromatography of this material through a Biotage silica cartridge (8 x 4 cm i.d.) using a gradient of hexane to 75% ethyl acetate (in hexane) afforded 147 mg (47% from *trans*-4-methylcyclohexylamine) of 426: rt=11.50 min.; m/z (rel. int.) 312 ($M^+$, 16), 216 (79), 200 (100), 172 (60), 142 (10), 114 (19), 89 (10), 87 (10).
In a similar manner, the following substituted quinoline-3-carboxamides were prepared:

**EXAMPLE 24: Preparation of *N*-(*trans*-4-methylcyclohexyl)-6,7-ethylenedioxyquinoline-3-carboxamide (427)**

Ethyl 4-chloro-6,7-ethylenedioxyquinoline-3-carboxylate was prepared using the methods of Erickson (J.Med. Chem.,1979, 22, 7, 816-823).

Hydrolysis of ethyl 4-chloro-6,7-ethylenedioxyquinoline-3-carboxylate (19.8 g, 66.2 mmol) afforded 13 g of crude 4-hydroxy-6,7-methylenedioxyquinoline-3-carboxylic acid hydrochloride.

The crude acid (500 mg), thionyl chloride, *trans*-4-methylcyclohexylamine hydrochloride (150 mg, 1 mmol), and triethylamine (0.3 mLs) afforded crude *N*-(*trans*-4-methylcyclohexyl)-4-chloro-6,7-ethylenedioxyquinoline-3-carboxamide.

Dehalogenation of the crude amide was accomplished in 1:1 ethanol-acetic acid (100 mL) with 10% Pd on carbon (200 mg) at 60 p.s.i. $H_2$ for 24 hours (ambient temperature). Workup and chromatography through a Biotage silica cartridge (8 x 4 cm i.d.) using a gradient of hexane to 60% ethyl acetate (in hexane) afforded 40 mg (12% from *trans*-4-methylcyclohexylamine) of 427:

rt=12.58 min.; m/z (rel. int.) 326 ($M^+$, 18), 230 (100), 214 (96), 186 (55), 131 (13), 103 (11), 75 (8), 55 (8) 41 (10).

**EXAMPLE 25: Preparation of *N*-(*trans*-4-methylcyclohexyl)-6,8-difluoroquinoline-3-carboxamide (428)**

Ethyl 4-chloro-6,8-difluoroquinoline-3-carboxylate was prepared from 2,4-difluoroanilne (12.9 g, 100 mmol) using the methods of Erickson (J.Med.Chem.,1979, 22, 7, 816-823).

Hydrolysis of ethyl 4-chloro-6,8-difluoroquinoline-3-carboxylate afforded 10.5 g of crude 4-hydroxy-6,8-difluoroquinoline-3-carboxylic acid hydrochloride. The crude acid (2 g), thionyl chloride, *trans*-4-methylcyclohexylamine hydrochloride (748 mg, 5 mmol), and triethylamine (0.3 mLs) afforded crude *N*-(*trans*-4-methylcyclohexyl)-4-chloro-6,8-difluoroquinoline-3-carboxamide.

Dehalogenation of the crude amide was accomplished in 5:1 tetrahydrofuran-triethylamine (300 mL) with 10% Pd on carbon (2.25 g) at 60 p.s.i. $H_2$ for 4 hours (ambient temperature). Workup and chromatography through a Biotage silica cartridge (8 x 4 cm i.d.) using a gradient of hexane to 20% ethyl acetate (in hexane) afforded 150 mg (49% from *trans*-4-methylcyclohexylamine) of 428:

rt=9.66 min.; m/z (rel. int.) 304 ($M^+$, 14), 209 (100), 192 (84), 164 (76), 144 (22), 87 (7), 81 (12).

**EXAMPLE 26: Preparation of *N*-(*trans*-4-methylcyclohexyl)-5,7-difluoroquinoline-3-carboxamide (429)**

Ethyl 4-chloro-5,7-difluoroquinoline-3-carboxylate was prepared from 3,5-difluoroanilne (12.9 g, 100 mmol) using the methods of Erickson (J.Med.Chem., 1979, 22, 7, 816-823).

Hydrolysis of ethyl 4-chloro-5,7-difluoroquinoline-3-carboxylate afforded 9.6 g of crude 4-hydroxy-6,8-difluoroquinoline-3-carboxylic acid hydrochloride.

The crude acid (500 mg), thionyl chloride, *trans*-4-methylcyclohexylamine hydrochloride (250 mg, 1.67 mmol), and triethylamine (0.3 mLs) afforded crude *N*-(*trans*-4-methylcyclohexyl)-4-chloro-5,7-difluoroquinoline-3-carboxamide.

Dehalogenation of the crude amide was accomplished in 4:1 tetrahydrofuran-triethylamine (75 mL) with 10% Pd on carbon (300 mg) under a $H_2$ balloon for 35 min (ambient temperature). Workup and chromatography through a Biotage silica cartridge (8 x 4 cm i.d.) using a gradient of hexane to 30% ethyl acetate (in hexane) afforded 134 mg (26% from *trans*-4-methylcyclohexylamine) of 429:

rt=9.23 min.; m/z (rel. int.) 304 ($M^+$, 14), 209 (94), 192 (100), 164 (65), 144 (18), 137 (14), 81 (16).

**EXAMPLE 27: Preparation of *N*-(*trans*-4-methylcyclohexyl)-6-methoxy-7-fluoroquinoline-3-carboxamide (430)**

Ethyl 4-chloro-6-methoxy-7-fluoroquinoline-3-carboxylate was prepared from 3-fluoro-*p*-anisidine (14.1 g, 100 mmol) using the methods of Erickson (J. Med. Chem., 1979, 22, 7, 816-823).

Hydrolysis of ethyl 4-chloro-6-methoxy-7-fluoroquinoline-3-carboxylate afforded 13 g of crude 4-hydroxy-6-methoxy-7-fluoroquinoline-3-carboxylic acid hydrochloride.

The crude acid (1 g), thionyl chloride, *trans*-4-methylcyclohexylamine hydrochloride (150 mg, 1 mmol), and triethylamine (0.3 mLs) afforded crude *N*-(*trans*-4-methylcyclohexyl)-4-chloro-6-methoxy-7-fluoroquinoline-3-carboxamide.

Dehalogenation of the crude amide was accomplished in 1:1 tetrahydrofuran-dichloromethane (100 mL) with 10% Pd on carbon (300 mg) at 60 p.s.i. $H_2$ for 4 hours (ambient temperature). Workup and chromatography through a Biotage silica cartridge (8 x 4 cm i.d.) using a gradient of hexane to 40% ethyl acetate (in hexane) afforded 150 mg (47% from *trans*-4-methylcyclohexylamine) of 430:

rt=10.77 min.; m/z (rel. int.) 316 ($M^+$, 16), 221 (60), 220 (58), 204 (100), 176 (69), 161 (12), 133 (12), 101 (8), 81 (6), 55 (6), 41 (12).

In a similar manner, the following substituted quinoline-3-carboxamides were prepared:

**EXAMPLE 28: Preparation of *N*-(*trans*-4-methylcyclohexyl)-7-trifluoromethylquinoline-3-carboxamide (431)**

4-Hydroxy-7-trifluoromethyl-3-carboxylic acid (514 mg, 2 mmol), thionyl chloride, *trans*-4-methylcyclohexylamine hydrochloride (225 mg, 1.5 mmol), and triethylamine (2 mL) afforded crude product. Chromatography through a Biotage silica cartridge (8 x 4 cm i.d.) using a gradient of hexane to 40% ethyl acetate (in hexane) afforded 514 mg (92%) of *N*-(*trans*-4-methylcyclohexyl)-4-chloro-7-trifluoromethylquinoline-3-carboxamide:

rt=9.74 min.; m/z (rel. int.) 370 ($M^+$, 8), 275 (100), 258 (80), 230 (42), 203 (13), 81 (23).

The amide (253 mg, 0.68 mmol) in 3:1 tetrahydrofuran-dichloromethane (75 mL) containing 10% Pd on carbon (150 mg) was hydrogenated at 40 p.s.i. $H_2$ for 10 min (ambient temperature). Filtration of the reaction mixture and chromatography through a Biotage silica cartridge (8 x 4 cm i.d.) using a gradient of hexane to 20% ethyl acetate (in hexane) afforded 40 mg (12%) of 431:

rt=9.38 min.; m/z (rel. int.) 336 ($M^+$, 13), 317 (3), 241 (100), 224 (85), 196 (90), 176 (12), 169 (23), 81 (17).

**EXAMPLE 29: Preparation of *N*-(*trans*-4-methylcyclohexyl)-6-trifluoromethylquinoline-3-carboxamide (432)**

4-Hydroxy-6-trifluoromethyl-3-carboxylic acid (514 mg, 2 mmol), thionyl chloride, *trans*-4-methylcyclohexylamine hydrochloride (225 mg, 1.5 mmol), and triethylamine (2 mL) afforded crude *N*-(*trans*-4-methylcyclohexyl)-4-chloro-6-trifluoromethylquinoline-3-carboxamide:

m/z (rel. int.) 370 ($M^+$, 10), 351 (2), 335 (2), 275 (100), 258 (81), 230 (42), 203 (18), 97 (16), 81 (23).

The crude amide in 3:1 tetrahydrofuran-dichloromethane (100 mL) containing 10% Pd on carbon (250 mg) was hydrogenated at 20 p.s.i. $H_2$ for 4 hours (ambient temperature). Filtration of the reaction mixture and chromatography through a Biotage silica cartridge (8 x 4 cm i.d.) using a gradient of hexane to 30% ethyl acetate (in hexane) afforded 100 mg (20% from *trans*-4-methylcyclohexylamine) of 432:
rt=9.33 min.; m/z (rel. int.) 336 ($M^+$, 12), 317 (2), 241 (100), 224 (84), 196 (63), 176 (40), 169 (23), 81 (20).

**EXAMPLE 30: Preparation of *N*-(*trans*-4-methylcyclohexyl)-8-trifluoromethylquinoline-3-carboxamide (433)**

4-Hydroxy-8-trifluoromethyl-3-carboxylic acid (514 mg, 2 mmol), thionyl chloride, *trans*-4-methylcyclohexylamine hydrochloride (225 mg, 1.5 mmol), and triethylamine (2 mL) afforded crude *N*-(*trans*-4-methylcyclohexyl)-4-chloro-8-trifluoromethylquinoline-3-carboxamide:
m/z (rel. int.) 370 ($M^+$, 10), 351 (2), 335 (2), 275 (100), 258 (76), 230 (33), 210 (29), 203 (13), 81 (33).

The crude amide in 3:1 tetrahydrofuran-dichloromethane (100 mL) containing 10% Pd on carbon (250 mg) was hydrogenated at 20 p.s.i. $H_2$ for 15 min (ambient temperature). An additional quantity of Pd on carbon (250 mg) was added and the reaction hydrogenated at 20 p.s.i. $H_2$ for 15 min (ambient temperature). Filtration of the reaction mixture and chromatography through a Biotage silica cartridge (8 x 4 cm i.d.) using a gradient of hexane to 30% ethyl acetate (in hexane) afforded 35 mg (7% from *trans*-4-methylcyclohexylamine) of 433:
rt=9.83 min.; m/z (rel. int.) 336 ($M^+$, 14), 317 (2), 241 (100), 224 (80), 196 (64), 176 (40), 169 (23), 81 (20).

**EXAMPLE 31: Preparation of *N*-(*trans*-4-methylcyclohexyl)-6-fluoroquinoxaline-2-carboxamide (434)**

**Synthesis of *N*-(4-fluoro-2-nitrophenyl)alanine ethyl ester.**

Using the methods of Lumma (*J. Med. Chem.,* **1981,** *24*, 93-101), a suspension of DL-alanine ethyl ester hydrochloride (24.1 g, 157 mmol) in dichloromethane (200 mL) was treated with 16 mL of 10 N NaOH (160 mmol) and the mixture stirred 15 min. Anhydrous $MgSO_4$ was added and the mixture stirred until a thick paste formed. The dichloromethane was decanted from the aqueous paste and further dried over anhydrous $MgSO_4$. The organic solution was filtered, and concentrated to approximately 50 mL in volume. This was added to a solution of 2,5-difluoronitrobenzene (25 g, 157 mmol) in toluene (50 mL). The reaction was heated at reflux until the total volume was less than 50 mL. A reflux condenser was added to the reaction flask and the solution heated at reflux for 2 hours. After this time, triethylamine (22 mL, 158 mmol) was added and the reaction heated at reflux for an additional 1 hour. The reaction mixture was then cooled, diluted with chloroform and adsorbed on to silica. Chromatography through silica (20 x 4.5 cm i.d.) using a gradient of hexane to ethyl acetate afforded 17.4 g (43%) of *N*-(4-fluoro-2-nitrophenyl)alanine ethyl ester:
rt=8.40 min; m/z (rel. int.) 256 (8, $M^+$), 183 (100), 137 (21), 122 (12), 109 (14), 95 (10), 83 (9).

**Synthesis of 7-fluoro-3-methyl-3,4-dihydro-2(1*H*)-ketoquinoxaline**

Using the methods of Lumma (*J. Med. Chem.*, **1981,** *24,* 93-101), a solution of *N*-(4-fluoro-2-nitrophenyl) alanine ethyl ester (17.4 g, 67.9 mmol) in ethanol was treated with mossy tin (35 g, 295 mmol) followed by concentrated HCl (75 mL). As the vigorous reflux subsided, the reaction was heated to, and maintained at reflux and for 1 hour. The solution was decanted from the remaining tin and concentrated to afford 3.69 g of crude 7-fluoro-3-methyl-3,4-dihydro-2(1*H*)-ketoquinoxaline:
rt=8.18 min; m/z (rel. int.) 180 ($M^+$, 29), 165 (42), 137 (100), 110 (17), 83 (15).

**Synthesis of 7-fluoro-3-methyl-2(1*H*)-quinoxalinone**

Using the methods of Lumma (*J. Med. Chem.*, **1981,** *24*, 93-101), the crude 7-fluoro-3-methyl-3,4-dihydro-2 (1*H*)-ketoquinoxaline (3.69 g) in ethanol (500 mL) was treated with 30% $H_2O_2$ (25 mL), and 1 *N* NaOH (50 mL) and the mixture heated at reflux overnight. After this time the reaction was cooled, acidified (pH ~3) with concentrated HCl, and the resulting solution extracted with chloroform (4 x 300 mL). The combined organic washes were dried over anhydrous $MgSO_4$, filtered, and concentrated to afford 3.2 g of crude 7-fluoro-3-methyl-2(1*H*)-quinoxalinone:
rt=8.42 min; m/z (rel. int.) 178 ($M^+$, 45), 150 (71), 149 (100), 122 (12), 108 (18).

**Synthesis of 2-chloro-3-methyl-7-fluoroquinoxaline**

Without purification, 3.2 g of crude 7-fluoro-3-methyl-2(1*H*)-quinoxalinone in phosphorous oxychloride (50 mL) was headed at 100 °C for 30 min, then poured over ice. The mixture was basified (pH ~10) with 10 *N* NaOH and

the resulting solution extracted with chloroform (4 x 300 mL). The combined organic washes were dried over anhydrous $MgSO_4$, filtered, and concentrated to afford 6.47 g of crude 2-chloro-3-methyl-7-fluoroquinoxaline:
rt=6.23 min; m/z (rel. int.) 198 ($M^+$, 13), 196 ($M^+$, 40), 161 (100), 134 (12), 120(20), 100 (12).

**Synthesis of 2-methyl-6-fluoroquinoxaline**

Without purification, 6.47 g of crude 2-chloro-3-methyl-7-fluoroquinoxaline, in a mixture of chloroform (200 mL), methanol (50 mL), and triethylamine (200 mL), was treated with palladium on carbon (2 g, 10% Pd) and stirred under a balloon of hydrogen for 45 min at ambient temperature. After this time the reaction was filtered and concentrated. Chromatography through a Biotage silica cartridge (8 x 4 cm i.d.) using a gradient of hexane to 20% ethyl acetate (in hexane) afforded 2.03 g (18% from of *N*-(4-fluoro-2-nitrophenyl)alanine ethyl ester) of 2-methyl-6-fluoroquinoxaline rt=5.24 min; m/z (rel. int.) 162 ($M^+$, 96), 135 (100), 94 (49).

**Synthesis of 6-fluoroquinoxaline-2-carboxaldehyde**

Using the method of Kepez (*Monatshefte fur Chemie*, **1989**, *120*, 127-130), a solution of 2-methyl-6-fluoroquinoxaline (225 mg 1.39 mmol) in ethyl acetate (30 mL) was treated with selenium dioxide (2.5 g) and the reaction mixture heated at reflux for 36 hours. The reaction was filtered hot and concentrated. Chromatography through a Biotage silica cartridge (8 x 4 cm i.d.) using a gradient of hexane to 10 % ethyl acetate (in hexane) afforded 71 mg (29 %) of 6-fluoroquinoxaline-2-carboxaldehyde.
rt=5.84 min; m/z (rel. int.) 176 ($M^+$, 92), 148 (76), 121 (100), 100 (33), 94 (63), 75 (17).

**Synthesis of 6-fluoroqainoxaline-2-carboxylic acid**

Using the method of Dodd (*Synthesis,* **1993,** 295-297), a solution of 6-fluoroquinoxaline-2-carboxaldehyde (71 mg, 0.4 mmol) in formic acid (2 mL) was cooled in an ice bath and treated with hydrogen peroxide (2 mL of 30 %). The reaction was stirred in the ice bath for 3 hours. After this time the reaction mixture was diluted with 10% HCl (25 mL) and extracted with dichloromethane (4 x 25 mL). The combined organic extracts were dried over anhydrous $MgSO_4$, filtered and concentrated to afford 35 mg (45%) of 6-fluoroquinoxaline-2-carboxylic acid.

***N*-(*trans*-4-methylcyclohexyl)-6-fluoroquinoxaline-2-carboxamide (434)**

A solution of 6-fluoroquinoxaline-2-carboxylic acid (35 mg, 0.18 mmol) in dimethylformamide (4 mL) was treated with 1,1'-carbonyldiimidazole (29 mg, 0.18 mmol) and the reaction stirred for 16 hours at ambient temperature. After this time the reaction mixture was treated with *trans*-4-methylcyclohexylamine hydrochloride (27 mg, 0.18 mmol) and *N,N*-diisopropylethylamine (0.05 mL, 0.29 mmol) and the reaction stirred 2 hours at ambient temperature. The solvent was evaporated to a solid (700 mg). The solid was dissolved in chloroform (10 mL) and added to diethyl ether (30 mL) The organic solution was washed with water (1 x 25 mL) and brine. The remaining organic solution was dried over anhydrous $MgSO_4$, filtered, and concentrated to a solid (35 mg) HPLC (10 micron silica, 250 x 20 mm i.d.) of this material using a gradient of chloroform to 10 % ethanol (in chloroform) afforded 20 mg (39%) of 434:
Rt=8.88 min; m/z (rel. int.) 287 ($M^+$, 23), 259 (1), 230 (9), 216 (3), 192 (22), 175 (36), 147 (100), 120 (28), 112 (47).

**EXAMPLE 32: *N*-(trans-4-methylcyclohexyl)-5-fluoroquinoxaline-2-carboxamide**

Using the procedures for compound 435 and those of Lumma (*J. Med. Chem*., **1981,** *24*, 93-101), Kepez (*Monatshefte fur Chemie,* **1989,** *120*, 127-130), and Dodd (*Synthesis*, **1993,** 295-297), 2,6-difluoronitrobenzene afforded compound 435:
Rt=8.85 min; m/z (rel. int.) 287 ($M^+$, 54), 259 (1), 230 (14), 216 (6), 192 (27), 175 (26), 147 (100), 127 (20), 121 (15), 120 (18), 112 (40).

**EXAMPLE 33: *N*-(trans-4-methylcyclohexyl)-6-trifluoromethylquinoxaline-2-carboxamide**

Using the procedures for compound 435 and those of Lumma (*J. Med. Chem.,* **1981,** *24,* 93-101), Kepez (*Monatshefte fur Chemie,* **1989,** *120,* 127-130), and Dodd (*Synthesis*, **1993,** 295-297), 4-fluoro-3-nitrobenzotrifluoride afforded compound 436:
Rt=8.64 min; m/z (rel. int.) 337 ($M^+$, 27), 318 (3), 280 (10), 242 (24), 225 (17), 197 (100), 170 (28), 112 (62).

**EXAMPLE 34: *N*-(trans-4-methylcyclohexyl)-6-methoxyquinoxaline-2-carboxamide**

Treatment of compound 434 (N-(trans-4-methylcyclohexyl)-6-fluoroquinoxaline-2-carboxamide) with a solution of sodium methoxide in methanol at ambient temperature afforded compound 347:
Rt= 10.26 min; m/z (rel. int.) 299 ($M^+$, 36), 271 (3), 242 (3), 228 (3), 203 (13), 187 (33), 159 (100), 117 (25), 112 (100).

**EXAMPLE 35: *N*-(trans-4-methylcyclohexyl)-6,7-methylenedioxyquinoxaline-2-carboxamide**

Using the method of Cai (*J. Med. Chem.*, **1997,** *40,* 730-738), commercially available (Sigma) benzo[1,3]dioxole-5,6-diamine and oxalic acid in 2 *N* HCl with heating (2.5 hours) will generate 6,7-methylenedioxy-3-methyl-2(1*H*)-quinoxalinone). Using the procedure for compound 435, treatment of 6,7-methylenedioxy-3-methyl-2(1*H*)-quinoxalinone) will afford 2-chloro-3-methyl-6,7-methylenedioxy-quinoxaline. Using the procedure for compound 435, catalytic hydrogenation of 2-chloro-3-methyl-6,7-methylenedioxy-quinoxaline will afford 3-methyl-6,7-methylenedioxyquinoxaline. Using the procedure for compound 435 and the method of Kepez (*Monatshefte fur Chemie,* **1989,** *120,* 127-130), oxidation of 3-methyl-6,7-methylenedioxyquinoxaline with selenium dioxide will afford 6,7-methylenedioxyquinoxaline-2-carboxaldehyde. Using the procedure for compound 435 and the method of Dodd (*Synthesis*, **1993,** 295-297), oxidation of 6,7-methylenedioxyquinoxaline-2-carboxaldehyde with formic acid and hydrogen peroxide will afford 6,7-methylenedioxyquinoxaline-2-carboxylic acid. Using the procedure for compound 435, activation of 6,7-methylenedioxyquinoxaline-2-carboxylic acid with 1 equivalent 1,1'-carbonyldiimidazole followed by treatment with 1 equivalent *trans*-4-methylcyclohexylamine hydrochloride and 1.5 equivalents *N,N*-diisopropylethylamine will afford crude product. Chromatography of this crude product through a Biotage silica cartridge, using a gradient of hexane to ethyl acetate and/or HPLC (10 micron silica, 250 x 20 mm i.d.) using a gradient of chloroform to 10 % ethanol (in chloroform) will afford purified product.

**EXAMPLE 36: Assay of mGluR Group I antagonist activity**

HEK-293 cells expressing a recombinant receptor as described in WO 97/05252 were loaded with 2 μM Fura-2 acetoxymethylester by incubation for 30-40 minutes at 37 °C in SPF-PCB (126 mM NaCl, 5 mM KCl, 1 mM $MgCl_2$, 20 mM Na-HEPES, 1.0 mM $CaCl_2$, 1 mg/mL glucose, and 0.5 % BSA, pH 7.4).

The cells were washed 1-2 times in SPF-PCB, resuspended to a density of 4-5 million cells/mL and kept at 37 °C in a plastic beaker. For recording fluorescent signals, the cells were diluted five-fold into a quartz cuvette with BSA-free 37 °C SPF-PCB to achieve a final BSA concentration of 0.1 % (1.2 mL of 37 °C BSA-free SPF-PCB + 0.3 mL cell suspension). Measurements of fluorescence were performed at 37 °C with constant stirring using a custom-built spectrofluorimeter (Biomedical Instrumentation Group, University of Pennsylvania). Excitation and emission wavelengths were 340 and 510 nm, respectively. To calibrate fluorescence signals, digitonin (Sigma Chemical Co., St. Louis, MO; catalog # D-5628; 50 μg/mL, final) was added to obtain maximal fluorescence ($F_{max}$), and the apparent minimal fluorescence ($F_{min}$) was determined by adding TRIS-Base/EGTA (10 mM, pH 8.3, final). Concentrations of intracellular $Ca^{2+}$ were calculated using a dissociation constant (Kd) of 224 nM and applying the equation:

$$[Ca^{2+}]_i = (F - F_{min} / F_{max}) \times Kd;$$

where F is fluorescence measured at any particular time of interest and F falls between $F_{max}$ and $F_{min}$.

Control responses to the addition of 5 mM $Ca^{2+}$ (final extracellular calcium concentration, 6 mM) were determined in separate cuvettes. Control responses to changes in extracellular calcium were determined throughout the length of the experiment. Compounds were tested at a single concentration per cuvette of cells, and all compounds were prepared in DMSO. Appropriate dilutions were made such that compounds were added in no greater volume than 10 μl per a total volume of 1500 μl (final DMSO not greater than 0.67%) to achieve any particular testing concentration.

Once a stable intracellular calcium baseline was achieved, the compound was added to the cuvette. The response or lack of response to the compound addition was allowed to stabilize for 1-3 minutes and then 5 mM calcium was added to determine the effect of the compound on the subsequent calcium response. Once the peak for the subsequent calcium response was obtained, digitonin and EGTA were added in a sequential manner to determine $F_{max}$ and $F_{min}$, respectively. Data were expressed as changes in intracellular calcium concentrations in nM. These changes in the calcium response post compound addition were compared to the control (no compound) calcium response. Responses to calcium in the presence of test compounds were normalized as a percent change from that of controls. Data were entered into a Levenberg-Marquardt analysis for non-linear least squares and an $IC_{50}$ and 95 % confidence intervals thereof were determined for each compound.

The invention thus has been disclosed broadly and illustrated in reference to representative embodiments described above. Those skilled in the art will recognize that various modifications can be made to the present invention without departing from the spirit and scope thereof.

## Claims

**1.** A compound having the structure,

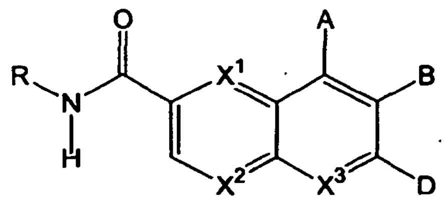

wherein $X^1$ and $X^2$ independently are CH or N,
$X^3$ is N or C-E
A, B, D, and E independently are selected from the group consisting of H, OMe, F, and $CF_3$, or B and D together are -O-($CH_2$)-O- or O-$(CH_2)_2$-O-, with the proviso that at least one of A, B, D and E is different from H,
R is selected from the group consisting of:

$C_4$-$C_6$ alkyl,

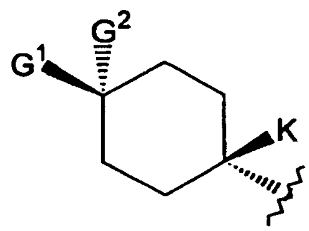

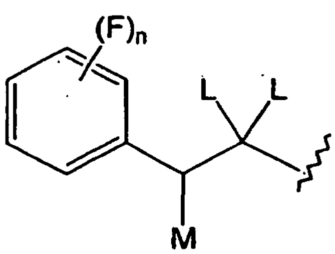 ,

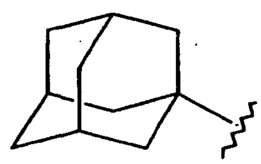 ,

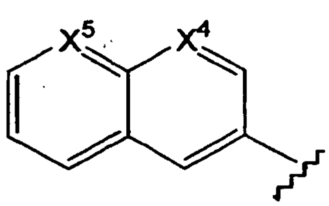 and 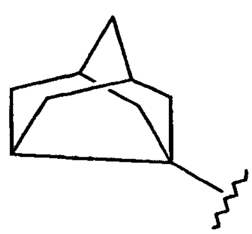

wherein K is H or Me,
$G^1$ is H, Me, or phenyl,

$G^2$, K, L, and M independently are H or Me,
n is 0, 1 or 2,
and $X^4$ and $X^5$ independently are N or CH, or a pharmaceutically acceptable salt thereof.

2. A compound according to claim 1, wherein $X^1$ is N and $X^2$ is N.

3. A compound according to claim 1, wherein $X^1$ is CH and $X^2$ is N.

4. A compound according to any of claims 1-3 selected from the group consisting of *N*-(*trans*-4-methylcyclohexyl)-6-methoxyquinoline-3-carboxamide, *N*-(*trans*-4-methylcyclohexyl)-7-methoxyquinoline-3-carboxamide, *N*-(*trans*-4-methylcyclohexyl)-5-fluoroquinoline-3-carboxamide, *N*-(*trans*-4-methylcyclohexyl)-7-fluoroquinoline-3-carboxamide, *N*-(*trans*-4-methylcyclohexyl)-6-fluoroquinoline-3-carboxamide, *N*-(*trans*-4-methylcyclohexyl)-8-fluoroquinoline-3-carboxamide, *N*-(*trans*-4-methylcyclohexyl)-6,7-methylenedioxyquinoline-3-carboxamide, *N*-(*trans*-4-methylcyclohexyl)-6,7-ethylenedioxyquinoline-3-carboxamide, *N*-(*trans*-4-methylcyclohexyl)-6,8-difluoroquinoline-3-carboxamide, *N*-(*trans*-4-methylcyclohexyl)-6-methoxy-7-fluoroquinoline-3-carboxamide, *N*-(*trans*-4-methylcyclohexyl)-7-trifluoromethylquinoline-3-carboxamide, *N*-(*trans*-4-methylcyclohexyl)-8-trifluoromethylquinoline-3-carboxamide, *N*-(*trans*-4-methylcyclohexyl)-6-fluoroquinoxaline-2-carboxamide, *N*-(*trans*-4-methylcyclohexyl)-5-fluoroquinoxaline-2-carboxamide, *N*-(*trans*-4-methylcyclohexyl)-6-trifluoromethylquinoxaline-2-carboxamide, *N*-(*trans*-4-methylcyclohexyl)-6-methoxyquinoxaline-2-carboxamide, *N*-(*trans*-4-methylcyclohexyl)-6,7-methylenedioxyquinoxaline-2-carboxamide, *N*-(*trans*-4-methylcyclohexyl)-8-methoxyquinoline-3-carboxamide, *N*-(*trans*-4-methylcyclohexyl)-5,7-difluoroquinoline-3-carboxamide, *N*-(*trans*-4-methylcyclohexyl)-6-trifluoromethylquinoline-3-carboxamide, and pharmaceutically acceptable salts thereof.

5. A pharmaceutical composition comprising a compound according to any of the preceding claims and a pharmaceutically acceptable diluent or excipient.

6. Use of an effective amount of a compound according to any of claims 1-4 for the preparation of a medicament for inhibiting activation of an mGluR Group I receptor.

7. Use of an effective amount of a compound according to any of claims 1-4 for the preparation of a medicament for inhibiting neural damage caused by excitatory activation of an mGluR Group I receptor.

8. Use of an effective amount of a composition according to claim 5 for the preparation of a medicament for treating a disease associated with glutamate induced neuronal damage.

9. Use according to claim 6, wherein said use is useful for the treatment of neurological disorders and diseases, psychiatric disorders and diseases, and ophthalmological disorders and diseases.

10. Use according to claim 9, wherein said neurological disorder and disease is selected from the group consisting of senile dementia, Parkinson's disease, Alzheimer's disease, Huntington's Chorea, pain, neuropathic pain, epilepsy, head trauma, anoxic injuries, ischemic injuries, and tinnitus.

11. Use according to claim 9, wherein said psychiatric disorder and disease is selected from the group consisting of schizophrenia, depression, and anxiety.

12. Use according to claim 9, wherein said ophthalmological disorders are selected from the group consisting of diabetic retinopathies and glaucoma.

## Patentansprüche

1. Verbindung mit der Struktur

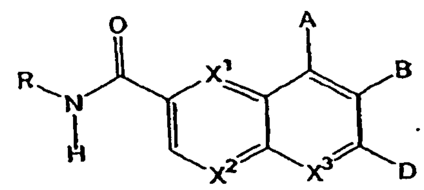

worin
$X^1$ und $X^2$ unabhängig CH oder N sind,
$X^3$, N oder C-E ist,
A, B, D, und E unabhängig ausgewählt sind aus der Gruppe, bestehend aus H, OMe, F, und $CF_3$, oder B und D zusammen $-O-(CH_2)_2-O-$ oder $O-(CH_2)_2-O-$ sind, mit der Maßgabe, dass mindestens eines aus A, B, D, und E von H verschieden ist,
R ausgewählt ist aus der Gruppe bestehend aus:

$C_4$-$C_6$-alkyl,

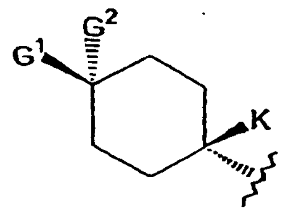

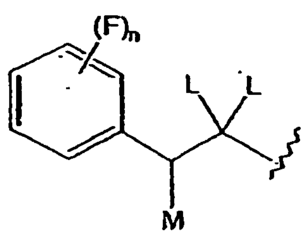

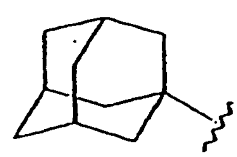

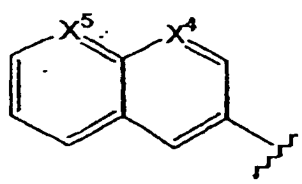

und

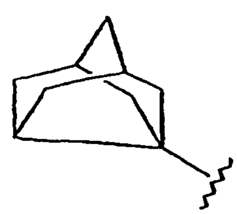

worin K H oder Me ist,
$G^1$ H, Me oder Phenyl ist,
$G^2$, K, L, und M unabhängig H oder Me sind,
n 0, 1 oder 2 ist
und $X^4$ und $X^5$ unabhängig N oder CH sind,
oder ein pharmazeutisch annehmbares Salz davon.

2. Verbindung nach Anspruch 1, worin $X^1$ N ist und $X^2$ N ist.

3. Verbindung nach Anspruch 1, worin $X^1$ CH ist und $X^2$ N ist.

4. Verbindung nach einem der Ansprüche 1-3, ausgewählt aus der Gruppe, bestehend aus N-(trans-4-Methylcyclohexyl)-6-methoxychinolin-3-carboxamid, N-(trans-4-Methylcyclohexyl)-7-methoxychinolin-3-carboxamid, N-(trans-4-Methylcyclohexyl)-5-fluorchinolin-3-carboxamid, N-(trans-4-Methylcyclohexyl)-7-fluorchinolin-3-carboxamid, N-(trans-4-Methylcyclohexyl)-6-fluorchinolin-3-carboxamid, N-(trans-4-Methylcyclohexyl)-8-fluorchinolin-3-carboxamid, N-(trans-4-Methylcyclohexyl)-6,7-methylendioxychinolin-3-carboxamid, N-(trans-4-Methylcyclohexyl)-6,7-ethylendioxychinolin-3-carboxamid, N-(trans-4-Methylcyclohexyl)-6,8-difluorchinolin-3-carboxamid, N-(trans-4-Methylcyclohexyl)-6-methoxy-7-fluorchinolin-3-carboxamid, N-(trans-4-Methylcyclohexyl)-7-trifluormethylchinolin-3-carboxamid, N-(trans-4-Methylcyclohexyl)-8-trifluormethylchinolin-3-carboxamid, N-(trans-4-Methylcyclohexyl)-6-fluorchinoxalin-2-carboxamid, N-(trans-4-Methylcyclohexyl)-5-fluorchinoxalin-2-carboxamid, N-(trans-4-Methylcyclohexyl)-6-trifluormethylchinoxalin-2-carboxamid, N-(trans-4-Methylcyclohexyl)-6-methoxychinoxalin-2-carboxamid, N-(trans-4-Methylcyclohexyl)-6,7-methylendioxychinoxalin-2-carboxamid, N-(trans-4-Methylcyclohexyl)-8-methoxychinolin-3-carboxamid, N-(trans-4-Methylcyclohexyl)-5,7-difluorchinolin-3-carboxamid, N-(trans-4-Methylcyclohexyl)-6-trifluormethylchinolin-3-carboxamid, und pharmazeutisch annehmbare Salze davon.

5. Pharmazeutische Zusammensetzung, die eine Verbindung nach einem der voranstehenden Ansprüche und ein pharmazeutisch annehmbares Verdünnungsmittel oder Exzipienz umfasst.

6. Verwendung einer wirksamen Menge einer Verbindung nach einem der Ansprüche 1-4 zur Herstellung eines Arzneimittels zur Inhibierung der Aktivierung eines mGluR-Gruppe I-Rezeptors.

7. Verwendung einer wirksamen Menge einer Verbindung nach einem der Ansprüche 1-4 zur Herstellung eines Arzneimittels zur Inhibierung einer neuralen Schädigung, die durch exzitatorische Aktivierung eines mGluR-Gruppe I-Rezeptors verursacht wird.

8. Verwendung einer wirksamen Menge einer Zusammensetzung nach Anspruch 5 zur Herstellung eines Arzneimittels zur Behandlung einer Krankheit, die mit Glutamat-induzierter neuronaler Schädigung verbunden ist.

9. Verwendung nach Anspruch 6, wobei die Verwendung zur Behandlung neurologischer Störungen und Krankheiten, psychiatrischer Störungen und Krankheiten und ophthalmologischer Störungen und Krankheiten nützlich ist.

10. Verwendung nach Anspruch 9, wobei die neurologische Störung und Krankheit ausgewählt ist aus der Gruppe, bestehend aus Dementia senile, Parkinson'scher Krankheit, Alzheimer'scher Krankheit, Chorea Huntington, Schmerz, neuropathischem Schmerz, Epilepsie, Kopftrauma, Anoxie-bedingter Schädigung, ischämischer Schädigung und Tinnitus.

11. Verwendung nach Anspruch 9, wobei die psychiatrische Störung und Krankheit ausgewählt ist aus der Gruppe, bestehend aus Schizophrenie, Depression und Angst.

12. Verwendung nach Anspruch 9, wobei die ophthalmologischen Störungen ausgewählt sind aus der Gruppe, bestehend aus diabetischen Retinopathien und Glaukom.

## Revendications

1. Composé ayant la structure,

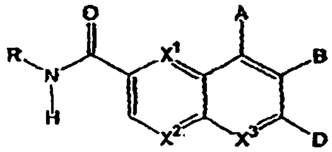

dans laquelle $X^1$ et $X^2$ sont, indépendamment, CH ou N,

$X^3$ est N ou C-E

A, B, D, et E sont, indépendamment, choisis dans le groupe composé de H, OMe, F, et $CF_3$, ou B et D ensemble sont $-O-(CH_2)-O-$ ou $O-(CH_2)_2-O-$, à condition qu'au moins un des A, B, D et E soit différent de H,

R est choisi dans le groupe composé de :

un alkyle $C_4$-$C_6$,

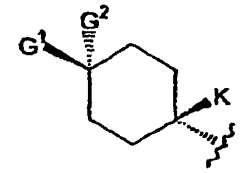

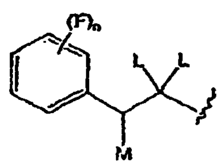

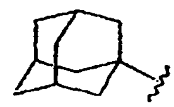

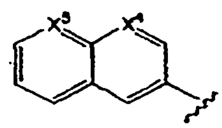

et

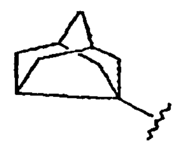

où K est H ou Me,
$G^1$ est H, Me, ou phényle,
$G^2$, K, L, et M sont indépendamment H ou Me,
n est 0, 1 ou 2,
et $X^4$ et $X^5$ sont, indépendamment, N ou CH,
ou sel pharmaceutiquement acceptable de celui-ci.

**2.** Composé selon la revendication 1, dans lequel $X^1$ est N et $X^2$ est N.

**3.** Composé selon la revendication 1, dans lequel $X^1$ est CH et $X^2$ est N.

**4.** Composé selon l'une quelconque des revendications 1 à 3 choisi dans le groupe composé du *N*-(*trans*-4-méthylcyclohexyl)-6-méthoxyquinoline-3-carboxamide, *N*-(*trans*-4-méthylcyclohexyl)-7-méthoxyquinoline-3-carboxamide, *N*-(*trans*-4-méthylcyclohexyl)-5-fluoroquinoline-3-carboxamide, *N*-(*trans*-4-méthylcyclohexyl)-7-fluoroquinoline-3-carboxamide, *N*-(*trans*-4-méthylcyclohexyl)-6-fluoroquinoline-3-carboxamide, *N*-(*trans*-4-méthylcyclohexyl)-8-fluoroquinoline-3-carboxamide, *N*-(*trans*-4-méthylcyclohexyl)-6,7-méthylènedioxyquinoline-3-carboxamide, *N*-(*trans*-4-méthylcyclohexyl)-6,7-éthylènedioxyquinoline-3-carboxamide, *N*-(*trans*-4-méthylcyclohexyl)-6,8-difluoroquinoline-3-carboxamide, *N*-(*trans*-4-méthylcyclohexyl)-6-méthoxy-7-fluoroquinoline-3-carboxamide, *N*-(*trans*-4-méthylcyclohexyl)-7-trifluorométhyl-quinoline-3-carboxamide, *N*-(*trans*-4-méthylcyclohexyl)-8-trifluorométhylquinoline-3-carboxamide, *N*-(*trans*-4-méthylcyclohexyl)-6-fluoroquinoxaline-2-carboxamide, *N* (*trans*-4-méthylcyclohexyl)-5-fluoroquinoxaline-2-carboxamide, *N*-(*trans*-4-méthylcyclohexyl)-6-trifluorométhylquinoxaline-2-carboxamide, *N*-(*trans*-4-méthylcyclohexyl)-6-méthoxyquinoxaline-2-carboxamide, *N*-(*trans*-4-méthylcyclohexyl)-6,7-méthylènedioxyquinoxaline-2-carboxamide, *N*-(*trans*-4-méthylcyclohexyl)-8-méthoxyquinoline-3-carboxamide, *N*-(*trans*-4-méthylcyclohexyl)-5,7-difluoroquinoline-3-carboxamide, *N*-(*trans*-4-méthylcyclohexyl)-6-trifluorométhylquinoline-3-carboxamide et sels pharmaceutiquement acceptables de ceux-ci.

**5.** Composition pharmaceutique comprenant un composé selon l'une quelconque des revendications précédentes et un diluant ou un excipient pharmaceutiquement acceptable.

**6.** Utilisation d'une quantité efficace d'un composé selon l'une quelconque des revendications 1 à 4 pour la préparation d'un médicament destiné à inhiber l'activation d'un récepteur de mGluR Groupe I.

**7.** Utilisation d'une quantité efficace d'un composé selon l'une quelconque des revendications 1 à 4 pour la préparation d'un médicament destiné à inhiber les lésions neurales provoquées par l'activation excitante d'un récepteur de

mGluR Groupe I.

8. Utilisation d'une quantité efficace d'une composition selon la revendication 5 pour la préparation d'un médicament destiné à traiter une maladie associée aux lésions neurales induites par le glutamate.

9. Utilisation selon la revendication 6, dans laquelle ladite utilisation est utile pour le traitement des troubles et des maladies neurologiques, des troubles et des maladies psychiatriques, et des troubles et maladies ophtalmologiques.

10. Utilisation selon la revendication 9, dans laquelle ledit trouble et maladie neurologique est choisi dans le groupe composé de la démence sénile, la maladie de Parkinson, la maladie d'Alzheimer, la chorée de Huntington, la douleur, la douleur neuropathique, l'épilepsie, le traumatisme crânien, les lésions anoxiques, les lésions ischémiques, et l'acouphène.

11. Utilisation selon la revendication 9, dans laquelle ledit trouble et maladie psychiatrique est choisi dans le groupe composé de la schizophrénie, la dépression, et l'anxiété.

12. Utilisation selon la revendication 9, dans laquelle lesdits troubles ophtalmologiques sont choisis dans le groupe composé des rétinopathies diabétiques et du glaucome.

# FIGURE 1 CONTINUED

| | |
|---|---|
| 408 | 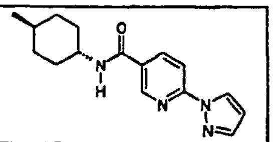 |
| 409 | 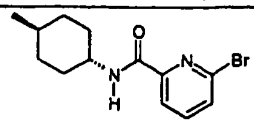 |
| 410 | 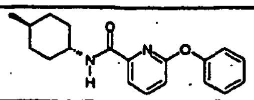 |
| 411 | 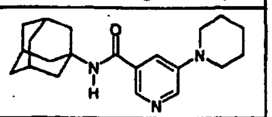 |
| 412 | 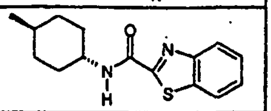 |
| 413 | 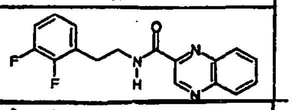 |
| 419 | 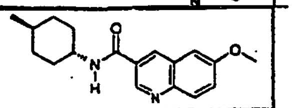 |
| 420 | 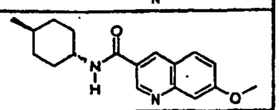 |
| 421 | 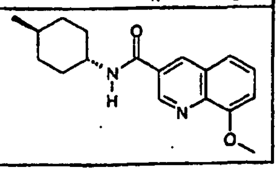 |

| | |
|---|---|
| 422 | 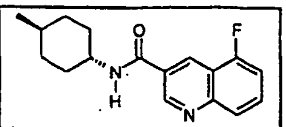 |
| 423 | 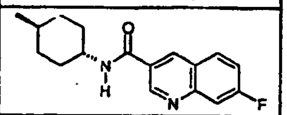 |
| 424 | 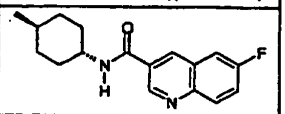 |
| 425 | 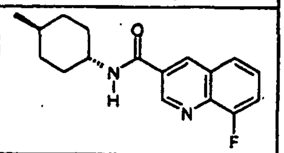 |
| 426 | 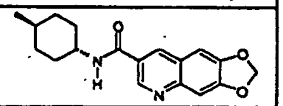 |
| 427 | 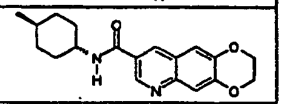 |
| 428 | 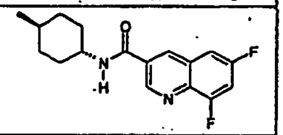 |
| 429 | 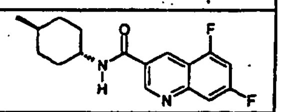 |
| 430 | 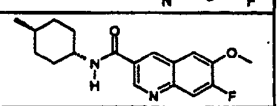 |
| 431 | 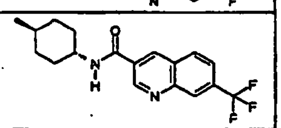 |
| 432 | 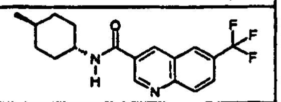 |
| 433 | 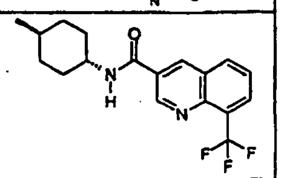 |
| 434 | 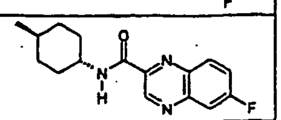 |
| 435 | 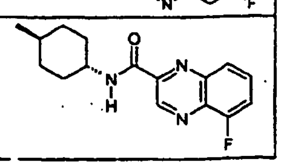 |

FIGURE 1 CONTINUED

| | |
|---|---|
| 436 | 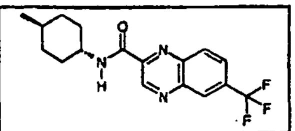 |
| 437 | 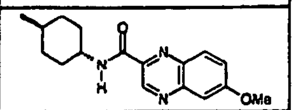 |
| 438 | 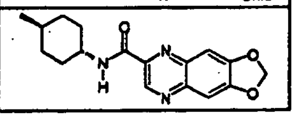 |